Europäisches Patentamt

European Patent Office

Office européen des brevets

(11) Publication number: **0 399 429**
**A1**

## EUROPEAN PATENT APPLICATION

(21) Application number: 90109583.6

(51) Int. Cl.⁵: **C12P 21/08, C12N 15/06**

(22) Date of filing: 21.05.90

The microorganism(s) has (have) been deposited with Fermentation Research Institute under number(s) BP-2878.

(30) Priority: 22.05.89 JP 128237/89
21.11.89 JP 304191/89

(43) Date of publication of application:
28.11.90 Bulletin 90/48

(84) Designated Contracting States:
BE DE ES FR GB IT NL

(71) Applicant: **TORAY INDUSTRIES, INC.**
**2-1, Nihonbashi Muromachi 2-chome**
**Chuo-ku**
**Tokyo 103(JP)**

(72) Inventor: **Ida, Nobuo 202, Toray Tebiro**
**Shataku, 1111**
**Tebiro, Kamakura-shi**
**Kanagawa 248(JP)**
Inventor: **Hosaka, Touko 4-4-4,**
**Minamitsukushino**
**Machida-shi**
**Tokyo(JP)**
Inventor: **Sakurai, Shingou**
**2-26-46,Bunkyo-cho**
**Mishima-shi**
**Shizuoka, 411(JP)**

(74) Representative: **Kador & Partner**
**Corneliusstrasse 15**
**D-8000 München 5(DE)**

(54) Anti-human interleukin-6 monoclonal antibody.

(57) An anti human interleukin-6 monoclonal antibody, which has a high specificity, as well as a hybridoma producing the same is disclosed. Not more than 4 ng of the monoclonal antibody can reduce the biological activity of 200 pg of human interleukin-6 by 50%.

EP 0 399 429 A1

## Anti-human Interleukin-6 Monoclonal Antibody

## BACKGROUND OF THE INVENTION

I. Field of the Invention

The present invention relates to an anti-human interleukin-6 monoclonal antibody which has a high neutralizing activity against the biological activity of human interluekin-6 (hereinafter referred to as "IL-6").

II. Description of the Related Art

Human IL-6 is a substance produced by various cells such as T cells, B cells, monocytes. fibroblasts and endothelial cells, which exhibits wide variety of biological activity such as differentiation of B cells to antibody-producing cells, stimulation of acute phase responses by hepatocytes, proliferation and differentiation of hematopoietic stem cells and differentiation of neurons (Ann. Rev. Immunol., 6, p485 (1988)).

The relationships between human IL-6 and various diseases are attracting attention. It is known that human IL-6 functions as an autocrine proliferation factor of myeloma (Nature 332, p83 (1988)), tumor cells of atrial myxoma produces a large amount of IL-6 (Proc. Natl. Acad, Sci. USA, 89 P228 (1987)), a high level of IL-6 exists in the synovial fluid and serum of chronic rheumatoid arthritis (Arthritis and Rheumatism. 31, p784 (1988), Eur. J. Immunol., 18, p1797 (1988)), and that when a rejection reaction occurs in a kidney-transplanted patient, the IL-6 level in the blood and urine increases in an early stage (Clin. Exp. Immunol. 71, p314 (1988)).

It is extremely useful in the study and the clinical diagnosis of these diseases to obtain an anti-human IL-6 monoclonal antibody and quantifying trace amount of IL-6 in the body fluid with the monoclonal antibody. Further, if the monoclonal antibody neutralizes the activity of IL-6, the monoclonal antibody may be used as a pharmaceutical for the treatment of these diseases.

From these view points, preparation of a monoclonal antibody to IL-6 has been tried. However, up to now, only two antibodies prepared by Matsuda et al are known (Eur. J. Immunol., 18, p951 (1988)). It was reported that one of these monoclonal antibodies had an activity of neutralizing the biological activity of IL-6. However, its neutralization activity is weak and 6 ng or more monoclonal antibody is required for reducing the biological activity (antibody production-inducing activity) of 0.1 ng of human IL-6. Further, as to the proliferation activity of IL-6-dependent hybridoma, even if 2 µg of the monoclonal antibody is used, no significant effect is observed on 0.2 ng of IL-6.

## SUMMARY OF THE INVENTION

Accordingly, the object of the present invention is to provide an anti-human IL-6 monoclonal antibody. which has a strong neutralization activity against the biological activity of human IL-6.

The present inventors intensively studied to succeed in obtaining an anti-human IL-6 monoclonal antibody with strong neutralization activity to complete the present invention.

That is, the present invention provides an anti-human interleukin-6 monoclonal antibody which can reduce the biological activity of 200 pg of human interleukin-6 by 50% with not more than 4 ng of the monoclonal antibody.

By the present invention, an anti-human IL-6 monoclonal antibody which specifically binds to human IL-6 with high neutralizing activity against the biological activity of human IL-6 was first provided. Since the neutralizing activity is high, in the diseases in which IL-6 is produced in an abnormally high level, such as myeloma, atrial myxoma and autoimmune diseases such as chronic rheumatoid arthritis, the biological activity of IL-6 increased by the abnormal production thereof may be reduced. Thus, it is expected that the monoclonal antibody of the present invention can be used as an extremely effective pharmaceutical for the treatment of these diseases. Further, by employing the monoclonal antibody of the present invention, an immunoassay system by which human IL-6 can be quantified easily with high sensitivity, as well as an immonoaffinity purification method may be provided.

## BRIEF DESCRIPTION OF THE DRAWINGS

Fig. 1 shows the results of the measurements of neutralizing activity of monoclonal antibodies against the biological activity of human IL-6 of different levels;

Fig. 2 shows the results of the measurements of neutralizing activity of monoclonal antibodies of various levels against the biological activity of a constant level of human IL-6;

Fig. 3 shows the results of the analysis of the peptide fragment which specifically binds to monoclonal antibody IG61 of the present invention;

Fig. 4 shows an inhibition effect of a synthetic peptide against an antibody-antigen reaction; and

Fig. 5 shows the peptide level dependency of the inhibition by the synthetic peptide.

## DETAILED DESCRIPTION OF THE PREFERRED EMBODIMENTS

As stated above, the anti-human IL-6 monoclonal antibody of the present invention has a strong neutralizing activity against the biological activity of human IL-6. More particularly, the monoclonal antibody of the present invention can reduce the biological activity of 200 pg of human interleukin-6 by 50% with not more than 4 ng, preferably not more than 2.5 ng of the monoclonal antibody.

The monoclonal antibody of the present invention may be prepared by hybridizing an antibody-producing cell of a mammal immunized with human IL-6 and a myeloma cell, selecting a hybridoma which produces an anti-human IL-6 antibody, cloning the hybridoma, culturing the hybridoma and recovering the anti-human IL-6 antibody from the culture. More particularly, the monoclonal antibody of the present invention may be prepared by the following steps:

(a) Preparation of Antibody-producing Cell

Firstly, a mammal is immunized with human IL-6. Either the natural IL-6 obtained from a cell originating from human or IL-6 prepared by a genetic recombination technique may be used.

The immunization may be performed by the conventional way. That is, the immunization may be carried out by intraperitoneal, subcutaneous, intracutaneous, intravenous injection or the like.

Mammals which are generally employed in the preparation of the monoclonal antibodies, such as mouse, rat, rabbit and guinea pig may be employed as the mammal to be immunized. In cases where a mouse is used as the mammal, human IL-6 may preferably be administered in the dose of 5 - 50 μg/mouse with or without a conventional adjuvant. The immunization is preferably repeated several times with an interval of at least one week. After 3 - 4 days from the final immunization, spleen cells or lymphocytes are collected and used as the antibody-producing cells in the subsequent steps.

(b) Cell Hybridization

The cell hybridization may be carried out in accordance with the conventional method well-known in the art, for example, in accordance with the method by Milstein et al (Method Enzymol., 73, p3 (1981)).

As the myeloma cells, well-known cells such as P3-X63-Ag8, P3-X63-Ag8-U1, SP20-Ag14, X63•Ag8-6.5.3 and the like may be employed.

The mixing ratio of the antibody-producing cells to myeloma cells may preferably be 5:1 - 10:1, and the cell hybridization may be carried out in the presence of a fusion promoter such as polyethyleneglycol (PEG) and Sendai virus (HVJ).

(c) Selection of Hybridoma with HAT Medium

After the cell hybridization step, the resulting cells may be suspended in a conventional medium used for cell culture such as RPMI1640 medium containing 15% bovine serum albumin, and the suspension is placed in each well in a microplate (usually 96-well type) in the amount of, for example, $10^5 - 10^6$ cells/100 μl/well. By adding HAT selection medium to each well and incubating the microplate for 7 - 14 days under the conditions well-known in the art, only hybridomas can be selectively grown.

3

In the selection step with HAT medium, to promote the efficiency of the formation of hybridomas and to promote the formation of antibody-producing hybridomas, it is preferred to add human or mouse IL-6 to the well in the level of 0.2 - 10 ng/ml. Further, since some hybridomas require IL-6 for their growing, until the desired antibody-producing cell is established as a single clone and until it is confirmed that the proliferation of the hybridoma does not depend on IL-6, it is preferred to always add human or mouse IL-6 to the wells.

(d) Screening

The screening of the desired antibody-producing cells may be carried out by any of the well-known methods such as enzyme linked immunosorbent assay (ELISA) employing the supernatant in each well as a sample. Further, for the confirmation of the more precise reaction specificity, Western blotting method may be employed.

(e) Cloning

From a positive well, a cell is isolated and cloned by the limiting dilution method, for example, so as to obtain a single clone producing the desired antibody. It is preferred that the cloning operation be repeated at least twice. The thus obtained hybridoma may be passaged in a conventional medium and also may be stored for a long period in liquid nitrogen.

(f) Obtaining Monoclonal Antibody

The monoclonal antibody may be obtained from the hybridoma by a conventional method. For example, the monoclonal antibody may be obtained by culturing the hybridomas and recovering the monoclonal antibody from the supernatant of the culture. Alternatively, the hybridoma may be administered intraperitoneally to an animal which is compatible with the hybridoma so as to grow the hybridoma in the animal and recovering the ascites from the animal.

In cases where the monoclonal antibody is obtained from the culture supernatant, a serum free medium (e.g., Celgrosser-H commercially available from Sumitomo Pharmaceuticals) may preferably be used in order to make the purification easy. The monoclonal antibody thus obtained may be purified by the conventional methods such as salting out, hydroxyapatite column chromatography, affinity chromatography and gel permeation chromatography.

(g) Selection of Antibody with Neutralizing Activity

The anti-human IL-6 monoclonal antibodies obtained by the method described above include those having neutralizing activity and those having no neutralizing activity. By subjecting the monoclonal antibodies to a measuring system of the biological activity of IL-6 to check their influence to the IL-6 activity, a monoclonal antibody with strong neutralizing activity may be selected.

As the measuring system of the biological activity of IL-6, known methods such as the method in which the induction of antibody production of B cells is measured (Proc. Natl., Acad. Sci. 82, p5490 (1985)), a method in which a hybridoma of which proliferation is dependent on IL-6 is used (J. Exp. Med., 165, p641 (1987)) may be employed.

[Examples]

Example 1

Preparation of Anti-human IL-6 Monoclonal Antibody

4

(a) Immunization of Animals

To 11 female balb/c mice of 7 - 9 weeks old, 5 - 15 $\mu$g/mouse of purified human IL-6 (prepared by the genetic recombination technique which was expressed in a baculovirus vector/insect cell system) was intraperitoneally administered.

The immunization was repeated 3 or 4 times with an interval of 1 - 10 weeks. In the first immunization, the administered formulation contained Freund's complete adjuvant or aluminum adjuvant containing dead *Bordetella pertussis*. In the second immunization, the administered formulation contained Freund's incomplete adjuvant or aluminum adjuvant. In the third or later immunization, the administered formulation contained PBS (8 mM disodium hydrogen phosphate, 1.5 mM potassium dihydrogen phosphate, 137 mM sodium chloride, 2.7 mM potassium chloride).

(b) After three days from the final immunization, spleen cells were separated from the mice. The spleen cells were mixed with mouse myeloma cells (P3-X63-Ag8-U1) with a mixing ratio of 10:1 in terms of the number of the cells, and the cell hybridization was carried out using 50% polyethylenglycol (1500) solution. After the cell hybridization, the cells were suspended in RPMI1640 medium containing 15% bovine serum albumin in the population density of about $2.5 \times 10^6$/ml in terms of the number of the spleen cells, and HAT and human IL-6 (final concentration of 2 ng/ml) were added to the suspension. The suspension was dividedly placed in each well of a 96-well microplate in the amount of 200 $\mu$l/well. After one week, hybridomas were grown in almost all of the wells.

(c) Screening of Antibody-producing Cells and Cloning Thereof

In each of the wells of a 96-well microplate, 50 mM sodium carbonate buffer containing 0.3 $\mu$g/ml of human IL-6 was placed in the amount of 100 $\mu$l each. After the microplate was left to stand overnight at 4°C, the solution was removed from the wells and the wells were blocked with a PBS solution containing 1% bovine serum albumin. After washing the wells with PBS containing 0.05% Tween 20 (PBS-T for short), 100 $\mu$l of the above-mentioned HAT selection culture supernatant (after 8 - 12 days from cell hybridization) was added to the each well and the reaction was carried out at room temperature for one hour. Subsequently, washing with PBS-T, reaction with biotin-labelled rabbit anti-mouse IgG at room temperature for 1 hour, washing with PBS-T, reaction with streptoavidin-horse radish peroxidase at room temperature for 0.5 hour, washing with PBS-T and reaction with 0.1 M phosphate citrate buffer (pH 4.0) containing 0.031% hydrogen peroxide and 0.1% ABTS at room temperature for 1 hour were carried out in the order mentioned in each well. The reaction was stopped with 100 $\mu$l each of 1% oxalic acid and the generated color of the reaction product was determined by measuring the absorbance at 414 nm. The cells which exhibited strong antibody activity were cloned by limiting dilution method. The above-mentioned screening and cloning procedures were repeated twice and 12 clones of antibody-producing hybridomas were established.

(d) Confirmation of Reaction Specificity

The culture supernatants of the 12 hybridomas obtained by the above-described screening employing ELISA were subjected to Western blotting analysis. As a result, it was confirmed that three clones specifically reacted with human IL-6. The monoclonal antibodies produced by these hybridomas were named IC67, IF14 and IG61, respectively. The hybridoma which produces IG61 was deposited with Fermentation Research Institute in Japan under Budapest Treaty under an accession number of FERM BP-2878.

(e) Obtaining Monoclonal Antibodies

The three hybridomas obtained as above were grown in abdominal cavities of balb/c mice and the ascites were collected therefrom. The monoclonal antibodies were purified according to a conventional method by salting out with 50% ammonium sulfate, hydroxyapatite column chromatography (eluate: sodium phosphate buffer 10 mM (pH 5.8) - 500 mM (pH 6.8) linear gradient) and Protein A column chromatography (equilibrated with 1.5 M glycine NaOH buffer (pH 8.9) containing 3M MaCl; eluate: 0.1 M citrate NaOH (pH 5.0).

Example 2

Determination of Subclass of Monoclonal Antibodies

The subclass of the three monoclonal antibodies were determined using mouse monoclonal antibody subclass-typing kit (commercially available from Zymed). As a result, the subclass of IC67, IF14, and IG61 was IgG·, IgM and IgG·, respectively.

Example 3

Measurement of Neutralizing Activities

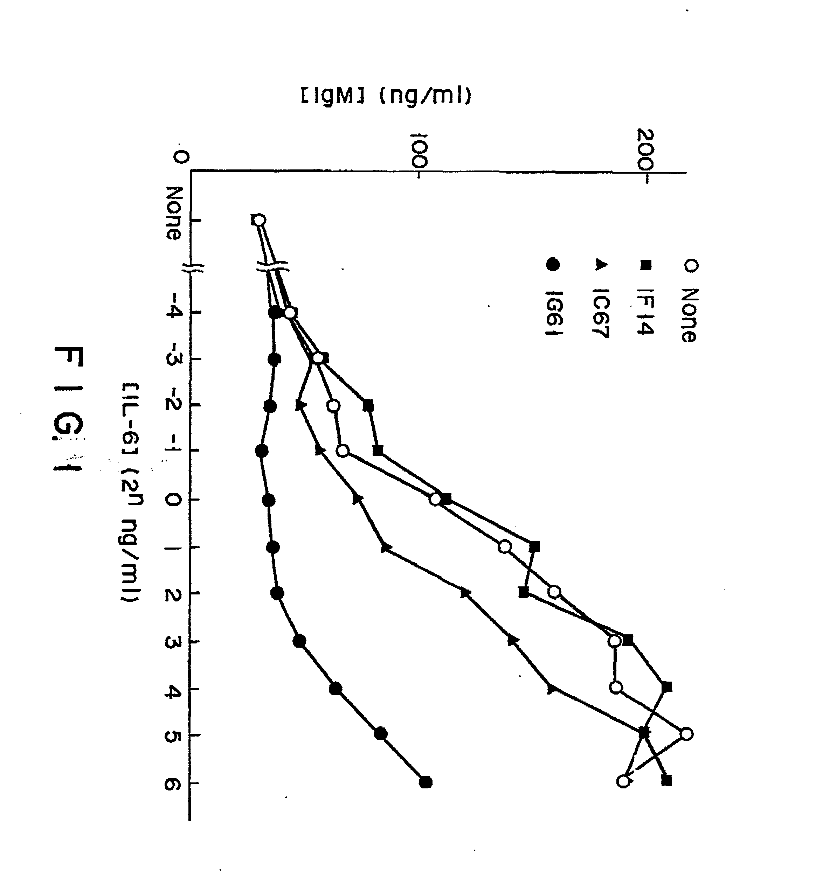

The biological activity of IL-6 was measured by using human B cell line SKW6-Cl-4 which produces IgM depending on IL-6 (Proc. Natl. Acad. Sci. USA, 82, P5490 1985)). A suspension of the SKW6-C1-4 cells ($4 \times 10^4$ cells/ml) in RPMI1640 medium was dividedly placed in each well of a 96-well microplate in the amount of 100 $\mu$l each). To each well, 50 $\mu$l of the monoclonal antibody with a level of 20 $\mu$g/ml and 50 $\mu$l of human IL-6 solution with various levels were added. After three days' incubation, 100 $\mu$l of supernatant was taken from each well and the level of IgM was determined by ELISA. The results are shown in Fig. 1. Among the three monoclonal antibodies, IG61 showed the strongest neutralizing activity against the biological activity of human IL-6, and the induction activity of antibody production of 64 ng/ml of IL-6 was reduced to that corresponding to 1 ng/ml of IL-6.

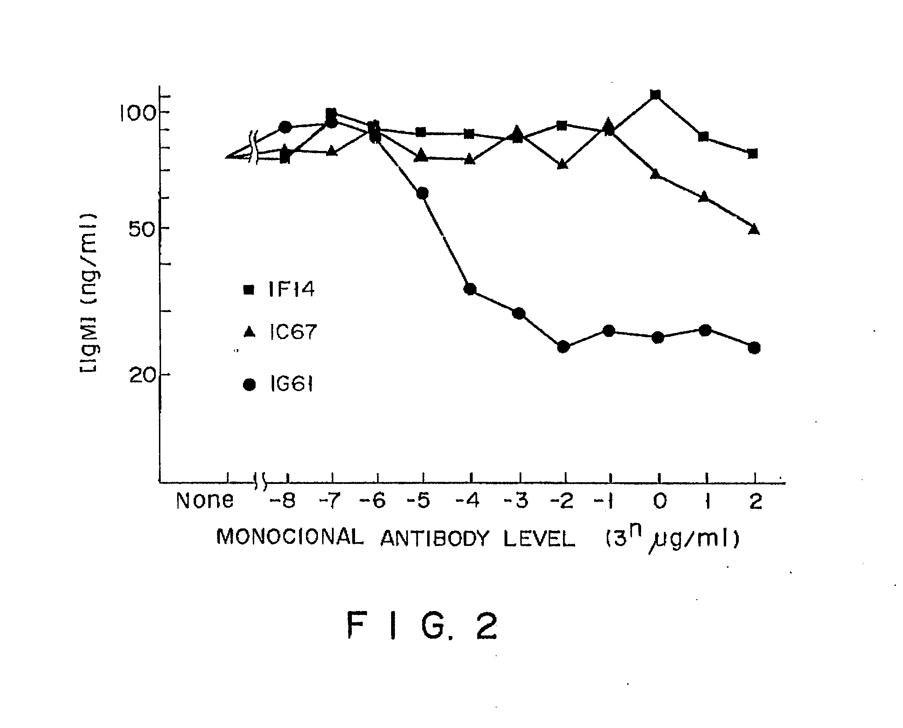

Further, the results of the experiments in which 50 $\mu$l of 4 ng/ml of human IL-6 and 50 $\mu$l of the monoclonal antibody with various concentrations were added to the same number of SKW6-1C-4 as above are shown in Fig. 2. It can be seen from Fig. 2 that the level of the IG61 of the monoclonal antibody of the present invention required for reducing the activity of 1 ng/ml of human IL-6 by 50% was about 10 ng/ml. In other words, the amount of the monoclonal antibody required for reducing the activity of 200 pg of human IL-6 by 50% was about 2 ng. The neutralizing activity of IG61 monoclonal antibody is much stronger than that of the anti-human IL-6 monoclonal antibody which has been reported.

Example 4

Analysis of Epitope

The epitope of the IG61 monoclonal antibody, on the human IL-6 molecule was analyzed as follows:

(1) Isolation and Analysis of Peptide Fragments Which React with Monoclonal Antibody

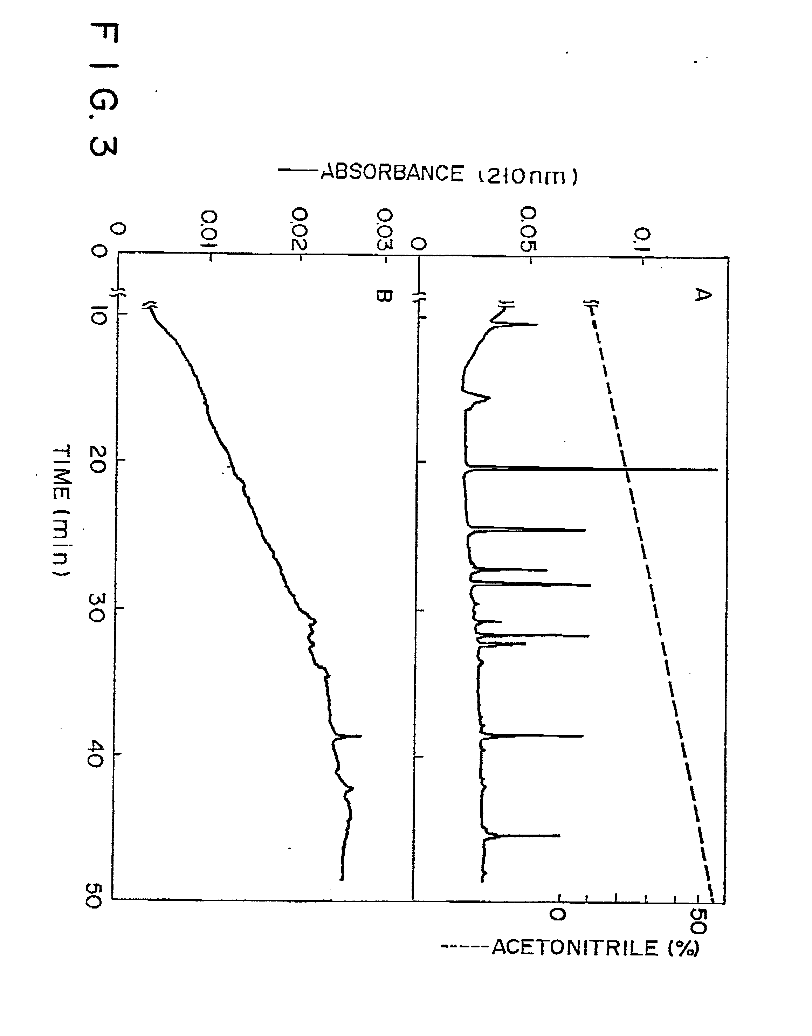

With 0.5 ml gel (Affigel 10 commercially available from Biorad), 0.5 mg of IG61 monoclonal antibody was reacted to prepare an antibody-immobilized gel. Twelve micrograms of human IL-6 was reacted with 120 ng of lysyl endopeptidase (commercially available from Wako Pure Chemicals) in 20 mM Tris-HCl buffer (pH 8.0). The reaction was carried out at 37°C for 6 hours to complete the digestion of the peptides. The digest was diluted to 1 ml and the resultant was adsorbed to the above-described IG61-immobilized gel at room temperature for 30 minutes. The gel after the peptide adsorption was packed in a small column and 5 ml of 0.1 M Tris-HCl buffer (pH 8.0) was passed through the column so as to wash off the non-reacted peptides. Thereafter, the adsorbed peptides were eluted with 0.1 M glycine-HCl (pH 2.4). A sample obtained after the treatment with lysyl endopeptidase but before the reaction with the antibody-immobilized gel and a sample which was a 0.5 ml - 1.0 ml fraction eluted with 0.1 M glycine-HCl (pH 2.4) were subjected to C18 reverse phase HPLC. The results are shown in Fig. 3A and 3B, respectively.

As a result of an analysis of the amino acid sequence of the peak (retention time 39 minutes) detected in Fig. 3B, a peptide with an amino acid sequence of Leu-Gln-Ala-Gln-Asn-Gln-Trp-Leu-Gln-Asp-Met-Thr-Thr-His-Leu-Ile-Leu-Arg-Ser-Phe-Lys which corresponds to 151st to 171st (from N-terminal) amino acid of human IL-6 was found.

. (2) Inhibition Effect of Antigen Antibody Reaction by Synthetic Peptides

A peptide IL-6 Thr149-Phe173 with 25 amino acids (Thr-Lys-Leu-Gln-Ala-Gln-Asn-Gln-Trp-Leu-Gln-Asp-Met-Thr-Thr-His-Leu-Ile-Leu-Arg-Ser-Phe-Lys-Glu-Phe) which contained the above-described peptide which was extended by two amino acids each in the directions of N-terminal and C-terminal on the human IL-6 molecule was synthesized and its influence on the EIA system was checked.

In each well of a 96-well microplate, 50 mM sodium carbonate buffer (pH 9.6) containing 0.3 µg/ml of human IL-6 was dividedly placed in the amount of 100 µl/well and the microplate was left to stand overnight at 4°C. Each well was blocked with PBS solution containing 1% BSA. After washing the each well with PBS-T, the synthetic peptide IL-6 Thr149 - Phe173 described above and a peptide containing 10 amino acids which had a sequence (Val-Gln-Ala-Ala-Ile-Asp-Tyr-Ile-Asn-Gly) with no relationship to the human IL-6 as a control, which had a level of 1mg/ml to 0.25 µg/ml in the amount of 50 µl each, and 50 µl of 0.1 µg/ml biotin-labelled IG61 antibody were placed in each well and the reaction was carried out at room temperature for 1 hour.

Subsequently, washing with PBS-T, reaction with streptoavidin-horseradish peroxidase at room temperature for 0.5 hour, washing with PBS-T and reaction with 0.1 M phosphate citrate buffer (pH 4.0) containing 0.031% hydrogen peroxide and 0.1% ABTS at room temperature for 1 hour were carried out in the order mentioned. The reaction was stopped with 100 µl each of 1% oxalic acid and the generated color of the reaction product was determined by measuring the absorbance at 414 nm. As a positive control, an experiment was conducted in the same manner except that human IL-6 was added in place of the synthetic peptide, and the inhibition effect was determined.

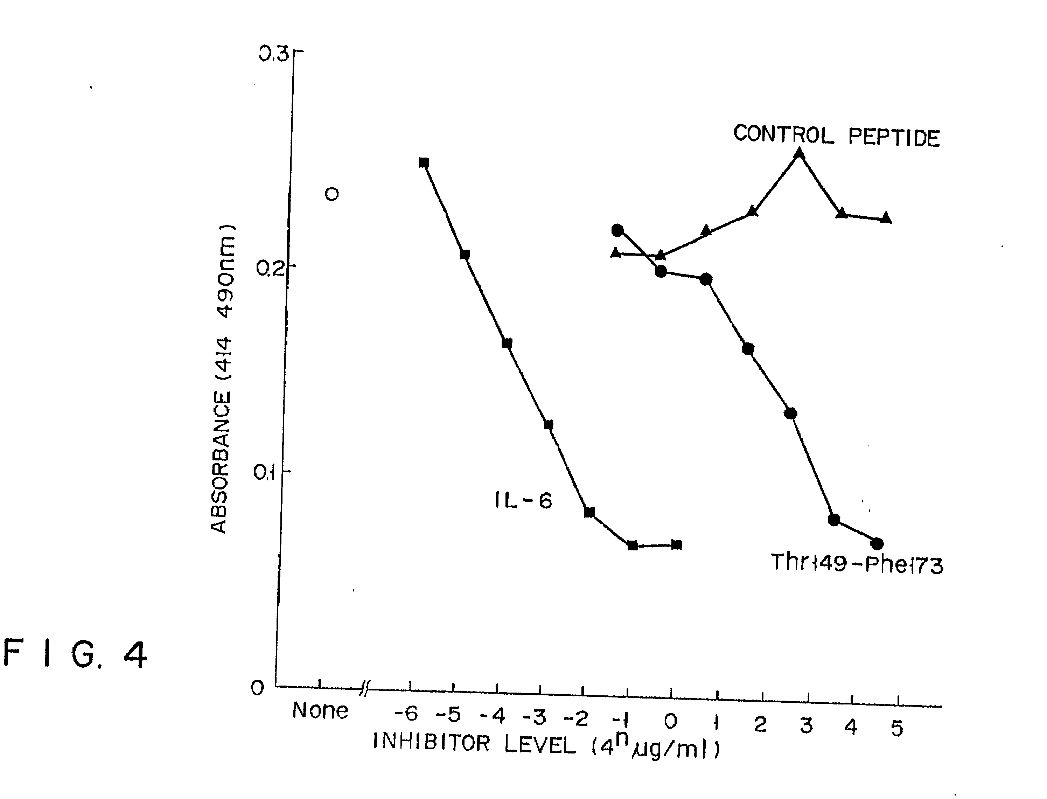

The results are shown in Fig. 4. As can be seen from Fig. 4, with the peptide Thr149-Phe173, the generation of color was decreased at a level of 8 µg/ml or more, and the reaction was completely inhibited at a level of 500 µg/ml. In contrast, the peptide as a control did not exhibit any inhibition effect.

(3) Detailed Analysis of the Inhibitory Effects with Synthetic Peptides on Antigen-Antibody Reaction

Eight peptides (peptide 1 - peptide 8, see Table 1, corresponding to the regions containing the 21-mer sequence in the IL-6 molecule (Leu151-Lys171), which was found in Example 4(1) were synthesized and were added to the EIA system of Example 4(2) to check the influence thereof.

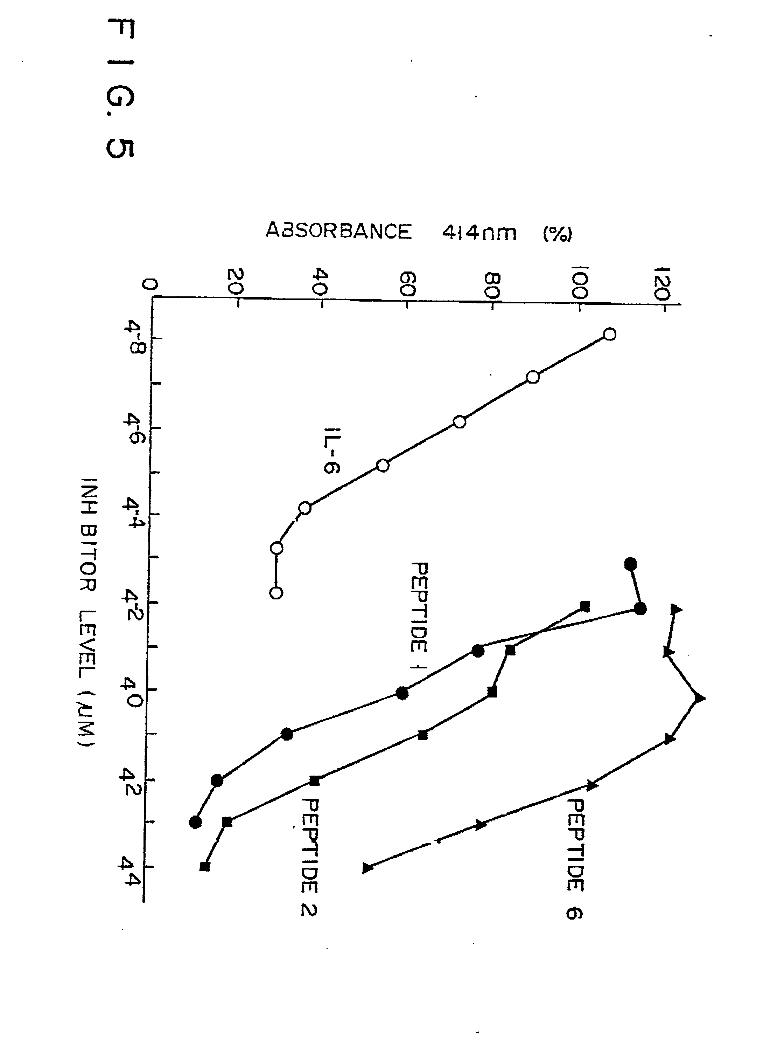

The results are shown in Table 1 and Fig. 5. The peptide 1 (Thr149-Phe173), peptide 2 (Ala153-Phe173) and peptide 6 (Ala153-Thr162) exhibited inhibition effect dose-dependently. Thus, it can be concluded that the region Ala153-Thr162 which is commonly contained in these peptides is the epitope of IG61 monoclonal antibody.

Table 1

| Peptide | Sequence | Inhibition Effect | IC50 (µM) |
|---------|----------|-------------------|-----------|
| Peptide 1 | TKLQAQNQWLQDMTTHLILRSFKEF | + + | 1.2 |
| Peptide 2 | QAQNQWLQDMTTHLILRSFKEF | + + | 6.5 |
| Peptide 3 | LQDMTTHLILRSFKEF | - | >100 |
| Peptide 4 | THLILRSFKEF | - | >100 |
| Peptide 5 | LLTKLQAQNQ | - | >500 |
| Peptide 6 | AQNQWLQDMT | + | 150 |
| Peptide 7 | DMTTHLILRL | | >500 |
| Peptide 8 | ILRSFKEFLQ | - | >500 |
| hrIL-6* | | + + + | 0.0004 |
| IL-6 sequence | [145]LLTKLQAQNQWLQDMTTHLILRSFKEFLQ[174]. | | |

* Human IL-6 prepared by genetic recombination technique

7

## Claims

1. An anti-human interleukin-6 monoclonal antibody which can reduce the biological activity of 200 pg of human interleukin-6 by 50% with not more than 4 ng of the monoclonal antibody.

2. The monoclonal antibody of claim 1, which can reduce the biological activity of 200 pg of human interleukin-6 by 50% with not more than 2.5 ng of the monoclonal antibody.

3. The monoclonal antibody of claim I, which belongs to class $IgG_1$.

4. The monoclonal antibody of claim 1, which binds to a region of 149th -173rd amino acid in the amino acid sequence of the human interleukin-6.

5. The monoclonal antibody of claim 4, which binds to a region of 153rd - 162nd amino acid in the amino acid sequence of the human interleukin-6.

6. The monoclonal antibody of claim 1, which is of any one of claims 1 - 6.

7. A hybridoma which produces the monoclonal antibody of any one of claims 1 - 6.

8. The hybridoma of claim 7, which has identifying characteristics of FERM BP-2878.

[IgM] (ng/ml)

200

100

0

None -4 -3 -2 -1 0 1 2 3 4 5 6

[IL-6] ($2^n$ ng/ml)

○ None
■ IF14
▲ IC67
● IG61

F I G. 1

F I G. 2

F I G. 3

EP 0 399 429 A1

# FIG. 4

ABSORBANCE (414  490nm)

INHIBITOR LEVEL ($4^n$ μg/ml)

None  -6  -5  -4  -3  -2  -1  0  1  2  3  4  5

IL-6

Thr149-Phe173

CONTROL PEPTIDE

# F I G. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-312996 (KISHIMOTO, TADAMITSU) * page 9, lines 1 - 42 * --- | 1-8 | C12P21/08 C12N15/06 |
| A | CLIN. EXP. IMMUNOL. vol. 75, no. 1, January 1989, Oxford, GB pages 93 - 99; N. JACKSON et al.: "Two new IgA1-k plasma cell leukaemia cell lines (JJN-1 & JJN-2) which proliferate in reponse to B cell stimulatory factor 2" ----- | | |

TECHNICAL FIELDS
SEARCHED (Int. Cl.5 )

C12P

The present search report has been drawn up for all claims

| Place of search | Date of completion of the search | Examiner |
|---|---|---|
| THE HAGUE | 23 JULY 1990 | NOOIJ F.J.M. |

EPO FORM 1503 03.82 (P0401)